# EUROPEAN PATENT APPLICATION

(11) **EP 4 501 285 A1**
(43) Date of publication of application: **05.02.2025**
(21) Application number: 23822972.8
(22) Date of filing: 06.06.2023
(51) Int. Cl.: A61F 2/24, A61B 17/12, A61M 31/00, A61F 2/966, A61F 2/95

(54) **VENTRICULAR FUNCTION ASSISTANCE DEVICE, DELIVERY AND RECOVERY SYSTEM, AND VENTRICULAR FUNCTION ASSISTANCE SYSTEM**

(30) Priority: 17.06.2022 CN 202210692170
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); HUANG, Haiyong, Shanghai 201203 (CN)
(74) Representative: Marks & Clerk LLP
(86) International application number: PCT/CN2023/098562
(87) International publication number: WO 2023/241403

(57) **Abstract**

The present invention relates to a ventricular function assisting device, a delivery and retrieval system and a ventricular function assisting system including the ventricular function assisting device and the delivery and retrieval system. The delivery and retrieval system includes a retrieval device and a delivery device. The retrieval device includes a constricting mechanism, and the delivery device includes a delivery rod and a delivery sheath. The delivery rod is configured to be releasably connected at its distal end to a base, and the constricting mechanism is configured to be releasably connected to a support structure. The delivery rod and the constricting mechanism can cooperate with each other to load the ventricular function assisting device into the delivery sheath. The ventricular function assisting device includes the support structure and the base. The base is configured to be releasably connected to the delivery rod. The support structure includes a plurality of backbones, all of which are arranged sequentially and circumferentially around the base. Each backbone is connected to the base at one end, and is free at the other end. Drug reservoirs are provided in outer surfaces of at least some of the backbones, thus drug utilization and long-term stability of adhesion are improved.

## Description

### TECHNICAL FIELD

The present invention relates to the field of medical devices, and particularly to a ventricular function assisting device, a delivery and retrieval system and a ventricular function assisting system.

### BACKGROUND

It is generally believed that heart failure with preserved ejection fraction (HFpEF) is currently the most prevalent heart failure disease type and HFpEF patients account for over 52% of the total heart failure population. Studies have found that HFpEF patients suffer from left ventricular concentric remodeling and myocardial stiffness and hypertrophy, which may lead to active diastolic dysfunction and hence an elevated left ventricular end diastolic pressure (LVEDP), increased resistance of blood flow from the left atrium into the left ventricle and increases in left atrial and pulmonary venous pressures as well. Consequently, HFpEF patients may develop symptoms such as dyspnea, acute lung congestion, digestive tract congestion and hydroperitoneum, which pose a huge threat to the lives of HFpEF patients.

At present, for HFpEF patients, except for sacubitril/valsartan (Entresto^{®}) tablets, there is no other medication proven to be capable of ameliorating their prognosis or reducing their mortality, despite some improvements in other endpoints, such as re-hospitalization, exercise tolerance and quality of life. Therefore, HFpEF treatment has been downgraded to treatment of comorbidities that affect the disease's progression. Currently available treatment options are mainly to reduce preload, treat complications, enhance exercise tolerance, relieve symptoms, establish chronic disease management and prevent re-hospitalization.

For heart failure, especially HFpEF, a potential therapy is to implant, into a patient's left ventricle, an assisting device capable of enhancing his/her heart's diastolic function. For example, there has been proposed a flower-like ventricular function assisting device for medical use, which includes two or more arms each including a bottom end, a free top end and an intermediate section extending between the ends. When the device is implanted inside the left ventricle, the part where its arms are connected is placed inside the ventricle at the apex of the heart on the endocardial surface, and its arms are bent upwardly relative to the base points such that the arms rest on the inner wall of the ventricle. The arms of the ventricular function assisting device are radially forced and bend toward each other during heart systole, and thereby store potential energy that is converted from kinetic energy of systolic motion of the ventricle, due to their elasticity. Correspondingly, during the heart diastolic, the arms of the device radially expand outward, applying an outward pressure on the inner wall of the ventricle, thereby assisting in the heart's diastole, at this time, the potential energy stored in the arms of the device is converted into kinetic energy. This ventricular function assisting device can be implanted by trans-apical surgery or percutaneous intervention into the heard of a HFpEF patient using a sheath and a delivery device, in order to enhance the patient's cardiac function by assisting the relaxation and filling of the left ventricle during diastole. However, during a percutaneous interventional procedure, when the implant is released to an inappropriate location, fails to sufficiently press against the wall due to a too small size, or is not successfully released, surgical retrieval may be necessary due to the absence of a corresponding retrieval device, which would cause significant trauma to the patient. In addition, after being implanted inside the left ventricle, the implant cannot firmly rest on the wall for a long time, and may displace over time as the heart contracts, twists and moves longitudinally. Although adhesion of the ventricular function assisting device to heart tissue can be improved by applying a biocompatible material, along with a coating and a drug capable of promoting tissue growth to the entirety or part of the arms, this may not only increase the implanted material, but may also adversely affect tissue growth due to possible undesired diffusion of the drug in all directions.

Therefore, it is necessary to further improve the conventional ventricular function assisting device to overcome at least one of its drawbacks as described above.

### SUMMARY OF THE INVENTION

It is an object of the present invention to provide a ventricular function assisting device, a delivery and retrieval system and a ventricular function assisting system, which facilitate cardiac relaxation, provide for targeted drug delivery and allow a drug to be implanted at a lower dose and utilized at a higher rate. In addition, the adhesion of the ventricular function assisting device and an inner ventricular wall and a long-term stability of adhesion are improved, while causing less damage to a patient.

At least one of the above objects is attained by a ventricular function assisting device provided in a first aspect of the present invention. The ventricular function assisting device comprises a folded configuration and an expanded configuration, and is switchable between the folded configuration and the expanded configuration, and comprises a support structure and a base. The base is configured to be releasably connected to a delivery device, and the support structure comprises a plurality of backbones which are arranged sequentially and circumferentially around the base, a first end of each backbone is connected to the base and the second end of each backbone is a free end, and outer surfaces of at least some of the backbones are provided with drug reservoirs..

Optionally, the drug reservoir may comprise a large-diameter hole and a small-diameter hole in communication with each other, the large-diameter hole having a larger diameter than the small-diameter hole, the large-diameter hole extending through an outer surface of the backbone, the small-diameter hole extending an inner surface of the backbone, the large-diameter hole configured to contain a drug therein.

Optionally, at least some of the backbones may comprise hollow areas, wherein at least some of the backbones are provided with the drug reservoirs in solid areas of the backbones outside the hollow areas.

Optionally, the hollow areas may be made up of single continuous hollow slots and/or a plurality of discontinuous hollow slots.

Optionally, the backbones may be in the form of sheets or bars, and a middle portion of the backbone may be formed with a single continuous hollow slot extending from a first end to a second end, and wherein the drug reservoir is provided along a solid area that is outside of the single continuous hollow slot.

Optionally, the backbones may be a mesh-like stent structure, wherein mesh openings in the mesh-like stent structure form the hollow slots, and the drug reservoirs are provided in surfaces of struts in the mesh-like stent structure.

Optionally, the mesh-like stent structure may be in the form of single-layer mesh sheet structure, comprising a plurality of wavy sections that are arranged circumferentially and side-by-side around the base, wherein, for each backbone, first ends of the wavy sections are joined and then connected to the base and second ends of the wavy sections are joined to form the free end of the backbone, and any pair of the wavy sections is connected by at least one deformable connecting struts.

Optionally, each wavy section may comprise a plurality of repetitive units, wherein each repetitive unit consists of a curved section and strut sections, wherein each end of the curved section is joined to the strut section, wherein each drug reservoir is provided in outer surface of the strut section and has a length smaller than or equal to a length of the strut section.

Optionally, each of the backbones may be provided at its free end with a threading hole, wherein the threading holes extend through the drug reservoirs, and/or are arranged independently from the drug reservoirs.

Optionally, the ventricular function assisting device may further comprise an anchor structure attached to the base and configured to connect target tissue.

Optionally, the anchor structure may comprise a plurality of spike-like structures extending toward the outside of the support structure, the spike-like structures are configured to pierce the target tissue. Alternatively, the anchor structure may comprise a helical structure configured to be screwed into the target tissue.

Optionally, a portion of the anchor structure, which is configured to be inserted into the target tissue, may be made of a biodegradable material.

Optionally, the base may be provided with a locking mechanism configured for snap engagement with the delivery device.

Optionally, the locking mechanism may comprise a plurality of elastic engagement elements, the plurality of elastic engagement elements are arranged sequentially and circumferentially around the base, and each elastic engagement element is configured to be retracted into the base under the action of an external force applied thereto and to extend out of the base due to its own elasticity when the external force is removed.

Optionally, the ventricular function assisting device may further comprise a drug structure that is provided in the drug reservoirs and comprised of a drug and a polymer carrier.

Optionally, each of the backbones may be elastic, and/or and end surface at the free end of each backbone may be a smooth curved surface.

Optionally, a maximum diameter of the support structure after expanded is greater than an inner diameter of a portion of a ventricle where the support structure presses against an inner wall of the ventricle, and thereby supporting the support structure on the inner wall of the ventricle by virtue of a stretchability thereof.

Optionally, the ventricular function assisting device may comprise three or four backbones, all of which are uniformly arranged circumferentially around the base.

At least one of the above objects is also attained by a delivery and retrieval system provided in a second aspect of the present invention, which is used to deliver and retrieve the ventricular function assisting device as defined above. The delivery and retrieval system comprises a retrieval device and a delivery device. The retrieval device comprises a constricting mechanism, and the delivery device comprises a delivery rod and a delivery sheath. The delivery rod is configured to be releasably connected at its distal end to the base of the ventricular function assisting device. The constricting mechanism is configured to be releasably connected to the support structure of the ventricular function assisting device. The delivery rod and the constricting mechanism are configured to cooperate with each other to load the ventricular function assisting device into the delivery sheath and/or to unload it from the delivery sheath.

Optionally, each of the backbones may be provided at its free end with a threading hole, wherein the constricting mechanism comprises a pull thread configured to be successively passed through all the threading holes of the backbones and extend axially within the delivery sheath, and wherein two ends of the pull thread extend beyond a proximal end of the delivery sheath and are fixed.

Optionally, the retrieval device may further comprise a retrieval catheter disposed in the delivery sheath, the retrieval catheter is arranged in parallel to the delivery rod within the delivery sheath, wherein the pull thread is passed through the retrieval catheter, and the retrieval catheter is provided at its proximal end with attachment structures, and the attachment structure is configured to fix two ends of the pull thread..

Optionally, the delivery rod and the retrieval catheter may be disposed in a single lumen or different lumens of the delivery sheath.

Optionally, the retrieval catheter may comprise two separate axially-extending lumens, wherein two sections of the pull thread are passed through two lumens, respectively.

Optionally, the delivery device may further comprise an additional catheter disposed in the delivery sheath, which is configured to be sleeved over the exterior of the delivery rod and thereby decouple the delivery rod from the base.

Optionally, the base may be provided with a locking mechanism, wherein the delivery rod is provided at its distal end with a connector configured for snap engagement with the locking mechanism.

Optionally, the connector may be a hollow tubular structure, and may be configured to be sleeved over the base.

Optionally, the delivery rod may be a coil spring formed by helically winding one or more wires. Alternatively or additionally, the delivery rod may have a different stiffness along an axial direction thereof, and an intermediate section of the delivery rod may be less stiff than its proximal and distal sections.

Optionally, the delivery sheath may have a flexible distal section located at a distal end, the flexible distal section is configured to receive the ventricular function assisting device therein.

At least one of the above objects is also attained by a ventricular function assisting system provided in a third aspect of the present invention, which comprises the ventricular function assisting device as defined above and the delivery and retrieval system as defined above.

At least one of the above objects is also attained by a method of implantation provided in the present invention, which comprises the steps of:
a) loading the ventricular function assisting device on the delivery rod in the delivery device and passing the pull thread of the retrieval device successively through the threading holes of the backbones in the ventricular function assisting device;
b) advancing both the delivery device and the retrieval device within the delivery sheath of the delivery device to a target site of a left ventricle; after reaching the target site, releasing the ventricular function assisting device and determining whether it is successfully released; and
c) if the ventricular function assisting device is successfully released, detaching the pull thread, decoupling the delivery rod from the ventricular function assisting device using the additional catheter, and finally withdrawing the delivery rod, the additional catheter and the like from the patient's body.

If the ventricular function assisting device is not successfully released, or is released at an inappropriate location, the ends of the pull thread are pulled, and the delivery rod of the delivery device is pushed forward, thereby narrowing a proximal opening of the ventricular function assisting device (defined by the free ends of all the backbones). Finally, the folded ventricular function assisting device is reloaded into the delivery sheath by manipulating the delivery rod in cooperation with the pull thread, and then optionally retrieved by retracting the delivery sheath.

In summary, the ventricular function assisting device of the present invention comprises a support structure and a base, wherein the base is configured to be releasably connected to a delivery device, the support structure comprises a plurality of backbones which are arranged sequentially and circumferentially around the base, a first end of each backbone is connected to the base and the second end of each backbone is a free end, and outer surfaces of at least some of the backbones are provided with drug reservoirs. With this arrangement, the ventricular function assisting device can be pressed against an inner wall of a stiff, non-compliant ventricle due to the stretchability of the backbones, thereby enhancing the function of the ventricle during diastole by allowing it to relax and fill properly. Moreover, a drug can be contained in the drug reservoirs in the outer surfaces of the backbones, instead of being coating on the inner and outer surfaces of the backbones, thus, the drug can be released from the drug reservoirs essentially toward the inner wall of the ventricle, providing for targeted delivery of the drug. This allows the drug to be loaded in a reduced amount and prevents its diffusion in all directions, which may adversely affect the growth of ventricular tissue. In addition to this, growth and spreading of ventricular tissue on the ventricular function assisting device can be facilitated, enhancing post-implantation adhesion of the ventricular function assisting device and the inner wall of the ventricle and long-term stability of the adhesion.

In the ventricular function assisting device of the present invention, at least some of the backbones comprise hollow areas. With this arrangement, ventricular tissue is further allowed to grow through the hollow areas into the interior of the support structure. This improves the adhesion of the ventricular function assisting device and the inner wall of the ventricle and long-term stability of the adhesion.

When the ventricular function assisting device is not successfully released, or is released at an inappropriate location, it can be reloaded and retrieved into the delivery sheath using the delivery and retrieval system of the present invention, instead of through a separate surgical procedure. Therefore, possible trauma to the patient can be reduced.

The ventricular function assisting device of the present invention is preferably snap-engaged with the delivery rod by the elastic engagement elements. With this arrangement, disengagement is achievable in an easier way, reducing surgical complexity. Moreover, compared with conventional threaded connection, the elastic engagement elements can be disengaged with less damage being caused by the anchor structure to the ventricular tissue, adding safety and reliability.

### BRIEF DESCRIPTION OF THE DRAWINGS

Those of ordinary skill in the art would appreciate that the following drawings are presented to enable a better understanding of the present invention and do not limit the scope thereof in any sense, in which:
Fig. 1a is a schematic structural view of a ventricular function assisting device according to a first embodiment of the present invention in a folded configuration;
Fig. 1b is an enlarged view of part *a* of the ventricular function assisting device of Fig. 1a;
Fig. 1c is a cross-sectional view taken along line A-A of Fig. 1b;
Fig. 2 is a schematic structural view of the ventricular function assisting device in the first embodiment of the present invention in a completely expanded configuration;
Figs. 3a to 3d illustrate how a base achieves locking and unlocking in the first embodiment of the present invention;
Fig. 4a is a schematic illustration of an anchor structure being attached to the base in the first embodiment of the present invention, showing backbones in a folded configuration;
Fig. 4b is an enlarged view of part *b* of Fig. 4a;
Fig. 5 is a schematic structural view of a delivery rod and a distal connector thereof in the first embodiment of the present invention;
Fig. 6 is a schematic illustration of a delivery rod in engagement with the ventricular function assisting device in the first embodiment of the present invention, showing the backbones in a folded configuration;
Fig. 7 is a schematic structural view showing the ventricular function assisting device and a retrieval device being both received in a delivery sheath in the first embodiment of the present invention;
Figs. 8a to 8c are simplified illustrations of the delivery sheath and a retrieval catheter in the first embodiment of the present invention;
Figs. 9a and 9b illustrates partial release (a) and complete release (b) of the ventricular function assisting device from a distal end of the delivery sheath in the first embodiment of the present invention, in which the complete release is accompanied by complete unfolding;
Figs. 10a to 10d are schematic diagrams showing decoupling of the delivery rod from the ventricular function assisting device (a), withdrawal of the delivery rod (b), withdrawal of an additional catheter (c) and withdrawal of a guidewire and the delivery sheath (d) in a process of withdrawing delivery and retrieval devices from a human body in the first embodiment of the present invention;
Fig. 11a is a schematic illustration of an anchor structure being attached to a base according to a second embodiment of the present invention, showing a support structure in a folded configuration;
Fig. 11b is a schematic structural view of part c of the anchor structure of Fig. 11a;
Fig. 11c is a schematic illustration of the anchor structure being attached to the base in the second embodiment of the present invention, showing the support structure in an expanded configuration;
Figs. 12a and 12b are schematic structural views of a ventricular function assisting device according to a third embodiment of the present invention, which includes four backbones (a) or three backbones (b); and
Fig. 13 is a schematic enlarged view of part of the ventricular function assisting device in the third embodiment of the present invention, in which d indicates the location of one of drug reservoirs.

### List of Reference Numerals

10, support structure; 11, backbone; 101, wavy section; 102, connecting strut; 103, repetitive unit; 103a, curved section; 103b, strut section; 111, secured end; 112, backbone body; 113, free end; 114, hollow area; 115, drug reservoir; 115a, large-diameter hole; 115b, small-diameter hole; 20, base; 21, inner bore; 22, locking mechanism; 221, elastic engagement element; 201, receptacle; 23, ventricular tissue; 24, inner ventricular wall; 26, guidewire; 110, delivery rod; 130, connector; 131, hollow cavity; 132, engagement slot; 150, delivery sheath; 151, flexible distal section; 30, anchor structure; 31, spike-like structure; 32, anchor bore; 33, helical structure; 210, pull thread; 220, retrieval catheter; 221, second rapid exchange port; 160, first rapid exchange port; 230, additional catheter.

### DETAILED DESCRIPTION

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of' is generally employed in the sense of "at least one" and "at least two" is generally employed in the sense of "two or more". Additionally, the use of the terms "first" and "second" herein is intended for illustration only and is not to be construed as denoting or implying relative importance or as implicitly indicating the numerical number of the referenced items. Accordingly, defining an item with "first" or "second" is an explicit or implicit indication of the presence of one or at least two such items. The terms "one end" and "other end", as well as "proximal end" and "distal end", may be used herein to generally refer to corresponding end portions including corresponding endpoints, rather than only to the endpoints. As used herein, the terms "proximal end" and "distal end" may be used with respect to a ventricular function assisting device with one end inserted inside a human body and another end extending outside the body for manipulation. In this regard, when used in relation to a component, the term "proximal end" refers to a location thereon closer to the manipulation end of the ventricular function assisting device that extends outside the human body, and the term "distal end" refers to a location on the component, which is closer to the end of the ventricular function assisting device that is inserted within the human body and therefore farther away from the manipulation end of the ventricular function assisting device. The terms "proximal end" and "distal end" may also be used to describe a manual operation, or an operation conducted by hand. In this regard, when used in relation to a component, the term "proximal end" refers to a location thereon closer to the operator, and the term "distal end" refers to a location on the component, which is closer to the ventricular function assisting device and therefore farther away from the operator. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure. When used herein in relation to a longitudinal axis, the terms "radial", "radially", "transverse" and "transversely" refer to a direction perpendicular to the longitudinal axis, "axial" and "axially" refer to a direction parallel to the longitudinal axis, and "circumferential" and "circumferentially" refer to a direction surround the longitudinal axis.

In order to address at least one problem associated with the prior art, there is disclosed herein a ventricular function assisting device, which facilitates cardiac relaxation and allows a drug to be coated on the implant at a lower dose and utilized at a higher rate. Moreover, adhesion of the ventricular function assisting device to the inner wall of the ventricle and long-term stability of the adhesion are improved. There is also disclosed herein a delivery and retrieval system, which is capable of delivering and retrieving the ventricular function assisting device, with less damage being caused to an inner ventricular wall during the release and withdrawal of a delivery device therein. Moreover, surgical retrieval and redeployment of the ventricular function assisting device can be avoided, reducing trauma caused to a patient. There is also disclosed herein a ventricular function assisting system including the ventricular function assisting device and the delivery and retrieval system.

Preferred embodiments of the present invention are described below with reference to the accompanying drawings. Whenever there is no conflict, the embodiments disclosed herein and features thereof can complement or be combined with each other. As used herein, the term "releasable connection" refers to a separable connection, that is, the connections between structures can be unconnected. It is noted that a support structure constructed in accordance with the present invention can be folded and loaded into delivery sheath, and in this folded configuration, confined by the delivery sheath, lengthwise curved backbones of the support structure are straightened so as to extend almost linearly in the delivery sheath. Once the support structure is released from the delivery sheath, the backbones regain their original lengthwise curved shape.

### <Embodiment 1>

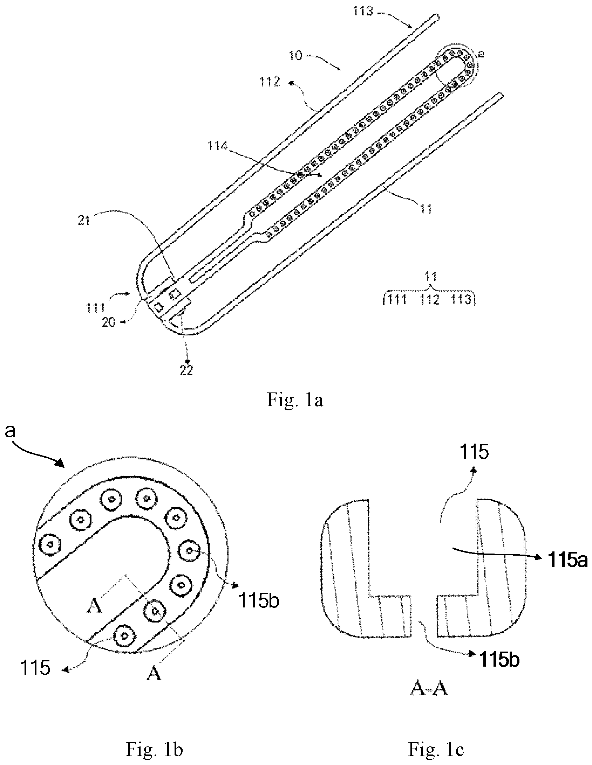

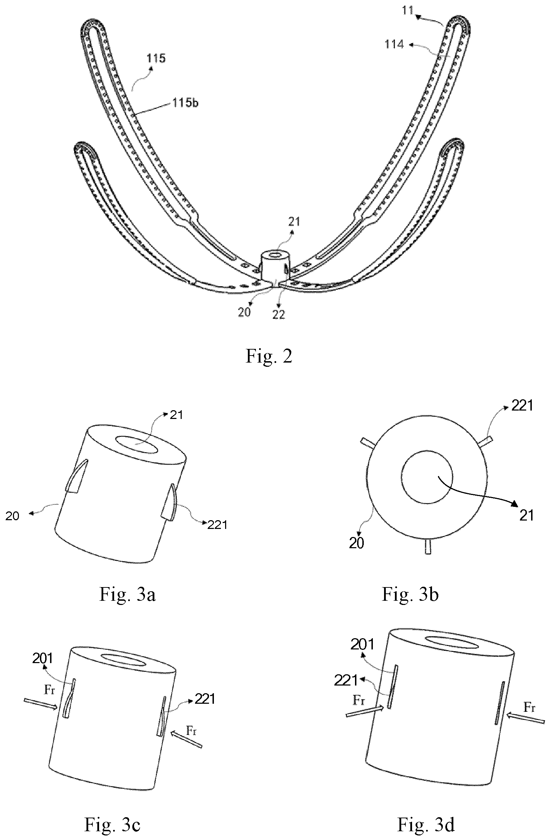

Figs. 1a to 1c show a ventricular function assisting device in a folded configuration according to a first embodiment of the present invention. Fig. 2 shows the ventricular function assisting device in an expanded configuration. As shown in Figs. 1 and 2, the ventricular function assisting device according to the first embodiment of the present invention is generally an umbrella-like structure, which is capable of comprising a folded configuration and an expanded configuration and is switchable between the folded and expanded configurations. Specifically, the ventricular function assisting device includes a support structure 10 and a base 20 for releasably connecting a delivery device. The support structure 10 is configured to, when expanded, be pressed against an inner ventricular wall to facilitate cardiac relaxation. The support structure 10 includes a plurality of backbones 11 together forming the aforementioned umbrella-like structure. At least two, preferably more than two, such as three, four or more, more preferably three or four backbones 11 may be included. Three or four backbones 11 can avoid an excessive additional burden on the heart, while providing sufficient diastolic support.

All the backbones 11 are arranged sequentially and circumferentially around the base 20, preferably uniformly. Each backbone 11 is attached to the base 20 at one end (referred to hereinafter as a secured end 111), for example, to a distal end of the base 20. In non-limiting examples, the attachment may be accomplished by welding, adhesive bonding, or the like. The other end of each backbone 11 is a free end 113. Each backbone 11 also has a backbone body 112 extending between the secured end 111 and the free end 113. In an implementation of application, the secured ends 111 of the backbones 11 are their distal ends, and the free ends 113 are their proximal ends. Preferably, each backbone 11 is elastic itself. That is, the support structure 10 is composed of elastic backbones. These enable the support structure 10 to expand by itself due to their own elasticity.

Fig. 1 shows the folded configuration, in which the backbone bodies 112 are gathered inwardly toward a longitudinal axis of the base 20 so that the ventricular function assisting device has a much smaller radial diameter, making the device appear like a collapsed umbrella, as a whole. This folded configuration facilitates stowing of the ventricular function assisting device, as a whole, within a delivery sheath of the delivery device. On the contrary, once the backbones 11 are released, their free ends 113 will deflect away from the longitudinal axis of the base 20. As a result, the ventricular function assisting device will have an expanded radial diameter, making the device comprise an expanded umbrella, as shown in Fig. 2. In the expanded configuration, the backbone bodies 112 each extend at an angle with respect to the longitudinal axis of the base 20, which may be greater than 90° and smaller than 180°, or smaller than or equal to 90°, depending on the anatomy of an inner ventricular wall, against which the backbone is to be pressed. Here, the angle is formed between the direction of extension of the backbone 11 and a positive direction of the longitudinal axis of the base 20, distal-to-proximal. The positive direction refers to a direction from a distal end to a proximal end of the base 20. The direction of extension may be a direction tangential to a surface of the backbone body 112, or a longitudinal axial direction of the backbone body 112 when the backbone body 112 itself is a straight structure. With this arrangement, the backbones 11 can be expanded to be pressed against the wall of the left ventricle, due to their own elasticity, facilitating cardiac relaxation and allowing a stiff, non-compliant ventricle to relax and fill properly.

Preferably, the backbones 11 are made of an elastic metal material for medical use, which enables them to expand by themselves. The present invention is not limited to any particular elastic metal material from which the backbones 11 are fabricated. It may be preferably a super-elastic material, more preferably an excellent biocompatible lightweight super-elastic material, even more preferably a shape memory alloy material, such as a nickel-titanium alloy. Each backbone 11, when expanded, has a smooth surface, which will not cause damage to an inner ventricular wall against which the backbone is to be pressed. In general, after expanded, the backbones 11 comprise a shape matching the anatomy of a target inner ventricular wall. Since such an inner ventricular wall tends to be curved, each backbone 11 may be expanded to comprise a curved contour along a lengthwise direction thereof, the curved section can be composed of either arcs with the same radius or arcs with different radii that are tangentially connected. It will be understood that each backbone 11 may adapt its own shape and size to a target inner ventricular wall so that it can be better pressed against the inner ventricular wall. Preferably, after expanded, the support structure 10 may have a maximum diameter greater than an inner diameter of a portion of the ventricular where the support structure 10 is pressed against the inner ventricular wall. For example, after expanded, free ends 113 of the backbones 11 may be uniformly located on a single circumference having a diameter greater than an inner diameter of a portion of the ventricular where the backbone 11 is pressed against the inner ventricular wall. In this way, the support structure 10 can be securely and firmly supported on the inner ventricular wall by virtue of their stretchability (elasticity).

Like a handle of the umbrella-like structure, the base 20 functions to connect both the support structure 10 and the delivery device. The base 20 comprises an inner bore 21 longitudinally extending therethrough, and a guidewire can be inserted through the inner bore 21 of the base 20. The present invention is not limited to any particular size or shape of the base 20, and it is preferred to be a hollow cylindrical structure. In order to allow a drug to be loaded in a smaller amount and utilized at a higher rate, drug reservoirs 115 are provided on the outer surfaces of at least some of the backbones 11, and a drug structure is contained in the drug reservoirs 115. The drug structure is comprised of a drug and a polymer carrier. A drug material can be formed by mixing the polymer carrier with the drug and then filled in the drug reservoirs 115 to form the drug structure. In addition to carrying the drug, the polymer carrier can also control release of the drug at a desired rate, thereby achieving its sustained release. The drug structure contained in the drug reservoirs 115 may form part of the ventricle assisting device. The drug reservoirs 115 may extend through both inner and outer surfaces of the backbones 11, forming through holes. Alternatively, they may extend through only the outer surfaces but not the inner surfaces, forming blind holes.

According to the present invention, the drug can be contained in the drug reservoirs 115 of the ventricular function assisting device, instead of being coated on the inner and outer surfaces of the backbones. With this arrangement, the drug in the drug reservoirs 115 is released essentially toward an inner ventricular wall. This allows for targeted delivery of the drug, which allows the drug to be loaded in a reduced amount and utilized at a higher rate. Additionally, the drug will not undesirably diffuse in all direction to unintended ventricular (or cardiac) tissue to affect growth thereof. Without such adverse influence on the growth of ventricular tissue, ventricular tissue can more easily grow and spread onto the ventricular function assisting device, enhancing post-implantation adhesion of the ventricular function assisting device to an inner ventricular wall and long-term stability of the adhesion. It will be understood that, according to the present invention, the drug is loaded only in the drug reservoirs 115 but not at any other location. In practice, the drug material may be precisely sprayed into the drug reservoirs 115 to form the drug structure.

The present invention is not limited to any particular drug, and examples of the drug may include, but are not limited to, those that promote tissue growth. In one embodiment, the drug may include at least one of an antithrombotic agent, an anticoagulant, an antiplatelet agent, an antitumor agent, an antiproliferative agent, an antibiotic, an anti-inflammatory agent, a genetic therapy agent, a recombinant DNA product, a recombinant RNA product, a collagen, a collagen derivative, a protein analog, a sugar, a sugar derivative, a smooth muscle cell proliferation inhibitor and an endothelial cell migration, proliferation and/or survival promoter, or a combination thereof. The present invention is not limited to any particular polymer carrier. For example, the drug-carrying matrix may be selected from poly(hydroxyalkanoate)s (PHA), poly(ester amide)s (PEA), poly(hydroxyalkanoate-co-ester amide), polyacrylates, polymethacrylates, polycaprolactones, polyethylene glycol)s (PEG), polypropylene glycol)s (PPG), polypropylene oxide) (PPO), polypropylene fumarate)s (PPF), poly(D-lactide)s, poly(L-lactide)s, poly(D,L-lactide)s, poly(meso-lactide)s, poly(L-lactide-co-meso-lactide)s, poly(D-lactide-co-meso-lactide)s, poly(D,L-lactide-co-meso-lactide)s, poly(D,L-lactide-co-PEG)s, poly(D ,L-lactide-co-trimethylene carbonate)s, poly(lactide-co-glycolide)s, poly(glycolic acid-co-trimethylene carbonate), poly(trimethylene carbonate)s, PHA-PEG, PBT-PEG (PolyActive^{®}), PEG-PPOPEG (Pluronict), PPF-co-PEG, polycaprolactones, polyglycerol sebacate, polycarbonates, biopolyesters, polyethylene oxides, polybutylene terephalates, poly(p-dioxanone)s, hybrids, complexes, collagen matrices with growth modulators, proteoglycans, glycosaminoglycans, vacuum formed small intestinal submucosa, fibers, chitin, dextran and combinations thereof.

The present invention is not limited to any particular shape of the drug reservoirs 115. For example, they may be in the shape of circular or elongate holes, or may have any other shape easy to machine. If required, any number of drug reservoirs 115 in any size may be provided, provided that the drug can be loaded in a sufficient amount, and it can be ensured that the backbones 11 have sufficient mechanical strength. In embodiments of the present invention, each backbone 11 is provided with a plurality of drug reservoirs 115 in its outer surface. The present invention is not limited to any particular number or distribution of drug reservoirs 115 on each backbone 11. Preferably, in order to achieve uniform release of the drug, the drug reservoirs 115 are arranged on the backbones 11 in a certain uniform pattern. In practical applications, the drug structure may be contained in some or all of the drug reservoirs 115, and users can choose the available drug reservoirs 115 and drug amount based on their practical needs. Preferably, each drug reservoir 115 includes a small hole extending through the inner surface of the backbone 11, which allows cardiac tissue to grow into and through the drug reservoir 115 and into the interior of the support structure 10. This further improves adhesion of the backbones 11 to the inner wall of the ventricle and long-term stability of the adhesion. Preferably, each drug reservoir 115 is stepped and composed of a large-diameter hole proximate the outer surface of the backbone 11 and a small-diameter hole proximate the inner surface of the backbone 11. The drug structure may be contained in the large-diameter holes.

Further, at least some of the backbones 11 may be each covered on their surfaces (including the inner and/or outer surfaces) with a non-drug-containing coating, which may be, for example, a coating capable of enhancing adhesion of the backbone 11 to an inner ventricular wall, or a chromium nitride coating capable of preventing the escape of some hazardous substances such as some harmful nickel ions, or a coating of a different function. For the purposes of a reduced weight, a reduced burden on the heart and easier spreading of cardiac tissue, at least some of the backbones 11 preferably comprise hollow areas 114, through which cardiac (or ventricular) tissue can grow. In these backbones 11, the drug reservoirs 115 are provided on the solid areas outside the hollow areas 114. With this arrangement, ventricular tissue can grow through the hollow area 114 into the interior of the support structure 10, improving adhesion of the backbones 11 to the inner wall of the ventricle and long-term stability of the adhesion.

Referring to Fig. 1a, in a specific embodiment, the hollow area 114 consists of continuous gaps, which minimize the weight of the support structure 10 and facilitate growth and spreading of cardiac tissue over larger areas. This more effectively improves adhesion of the backbones 11 to the inner wall of the ventricle and long-term stability of the adhesion. However, those skilled in the art will understand that, in other embodiments, each hollow area 114 may consist of multiple discontinuous hollow portions. For example, in embodiments of the present invention, each backbone 11 may be a sheet or bar comprising, in its middle portion, a single continuous hollow slot extending from one end to the other, and the drug reservoirs 115 are provided on a solid area outside of and surrounding the single continuous hollow slot.

Referring to Figs. 1b and 1c, each drug reservoir 115 is preferably stepped and includes a large-diameter hole 115a and a small-diameter hole 115b in communication with each other. The large-diameter hole 115a has a larger diameter than the small-diameter hole 115b. The large-diameter hole 115a extends through the outer surface of the backbone 11, and the small-diameter hole 115b extends through the inner surface of the backbone 11. The drug structure is contained in the large-diameter holes 115a. As a result of release of the drug from the drug reservoir, cardiac tissue can grow successively through the large-diameter holes 115a and the small-diameter holes 115b into the interior of the support structure 10. In this process, the small-diameter holes 115b can reduce leakage and release of the drug to the inner surfaces of the backbones 11.

Further, end surfaces of the free ends 113 of the backbones 11 are configured as non-damaging surfaces, such as smooth curved surfaces. Preferably, they are circularly curved surfaces, more preferably semicircular surfaces. With this arrangement, a smooth transition is provided at the end surfaces of the free ends 113 and the backbone bodies 112 (that is, a smooth and rounded corner is provided), reducing friction encountered by the ventricular function assisting device during its movement within a delivery sheath, which may cause damage to an inner wall of the delivery sheath and the backbones 11. Furthermore, the backbones 11 can be expanded to be more smoothly pressed against an inner ventricular wall, without causing damage to ventricular tissue.

When completely expanded, the free ends 113 of the backbones 11 in the ventricular function assisting device may be all located on a single circumference, or not. That is, the backbones 11 may have different lengths, or may be expanded to different extents. Preferably, the free ends 113 of the backbones 11 are angularly equidistantly located on a single circumference. In this way, the backbones 11 can be more uniformly pressed against an inner ventricular wall, providing for better cardiac relaxation. The support structure 10 may be expanded to have a maximum diameter, for example, defined by the free ends 113, greater than an inner diameter of a portion of a ventricle at full diastole. With this arrangement, during cardiac diastole, the backbones 11 can always push the inner wall of the ventricle. This, it can ensure the adhesion thereof and long-term stability of the adhesion.

Optionally, 3-10 backbones 11 may be included, each having a length of 25-65 mm and a thickness of 0.1-1.2 mm. These dimensions can accommodate the needs of most patients for ventricular function assistance. Preferably, each backbone 11 is provided at the free end 113 with a threading hole (not shown) configured to be releasably connected to a retrieval device. In one example, the threading holes are integrally formed with the drug reservoirs 115. That is, some of the drug reservoirs 115 may also serve as the threading holes. Without limitation, either one or more threading holes may be provided in each backbone 11. In another example, the threading holes are independent from the drug reservoirs 115. That is, the threading holes serve only to connect the retrieval device, while the drug reservoirs 115 serve only for drug loading. In other examples, some of the drug reservoirs 115 provide some of the threading holes, while the remaining threading holes are independent from the remaining drug reservoirs 115.

As noted above, the base 20 can be releasably connected to the delivery device. In a preferred embodiment, the base 20 is provided with a locking mechanism 22configured for snap engagement with the delivery device. Preferably, the locking mechanism 22 is an elastic locking mechanism, which allows for easier release and hence easier surgical operation and can reduce possible damage caused during release by an anchor structure 30 to ventricular tissue.

Referring to Figs. 3a to 3d, the elastic locking mechanism includes a plurality of elastic engagement elements 221, such as two, three or more elastic engagement elements 221, which are arranged sequentially and circumferentially around the base 20. Preferably, all the elastic engagement elements 221 are uniformly arranged circumferentially around the base 20. Each elastic engagement element 221 is stretchably mounted on the base 20, and the elastic engagement element 221 is to be retractable into the base 20 under the action of an external force applied thereto and extendable out of the base 20 when the external force is removed. For example, receptacles 201 for accommodating the elastic engagement elements 221 may be formed in a side wall of the base 20. Unlocking can be accomplished by retracting the elastic engagement elements 221 into the receptacles 201 under external force, and locking can be accomplished by extending the elastic engagement elements 221 out of the receptacle 201 due to their own elasticity when they are not pressed anymore. The elastic engagement elements 221 may be of various shapes, such as tabs, studs, blocks, etc., and the present invention is not limited to any particular shape of them. In a specific embodiment, the elastic engagement elements 221 are each shaped like one half of a circular segment, and are arranged uniformly on a side face of the base 20. In more detail, as shown in Figs. 3a and 3b, in a non-stressed, the elastic engagement elements 221 extend out of the receptacles 201. As shown in Figs. 3c and 3d, when radially inward compression forces Fr are applied to the respective elastic engagement elements 221, they will be gradually retracted back into the receptacles 201 from the extended configuration.

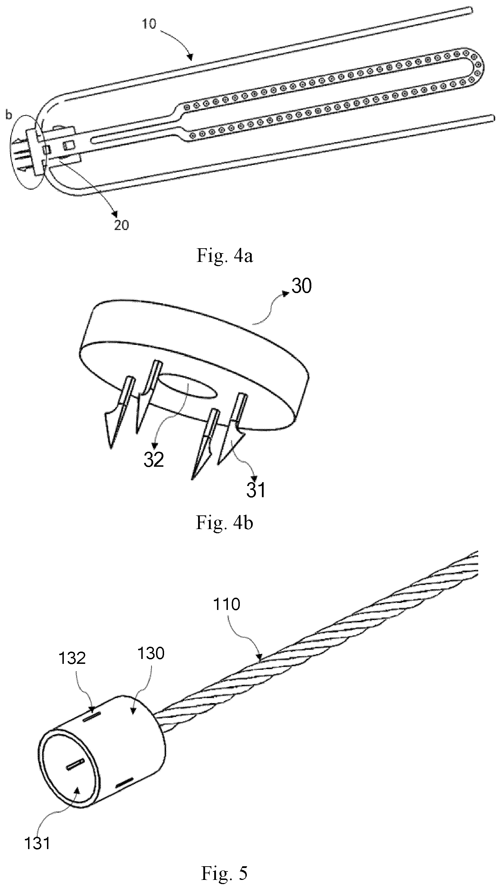

Embodiments of the present invention also provide a delivery and retrieval system for delivering and retrieving the ventricular function assisting device. Referring to Fig. 5, the delivery and retrieval system includes a delivery device for delivering the ventricular function assisting device to and releasing it at a target site. The delivery device includes a delivery rod 110 configured to be releasably connected to the base 20 at a distal end thereof. The releasable connection may be accomplished, for example, by a threaded connection, snap engagement or other mechanical or non-mechanical connection, with a snap engagement being preferred. In a specific embodiment, the delivery rod 110 includes a distal connector 130 at a distal end thereof, and the connector 130 is configured for snap engaging the locking mechanism 22 of the base 20.

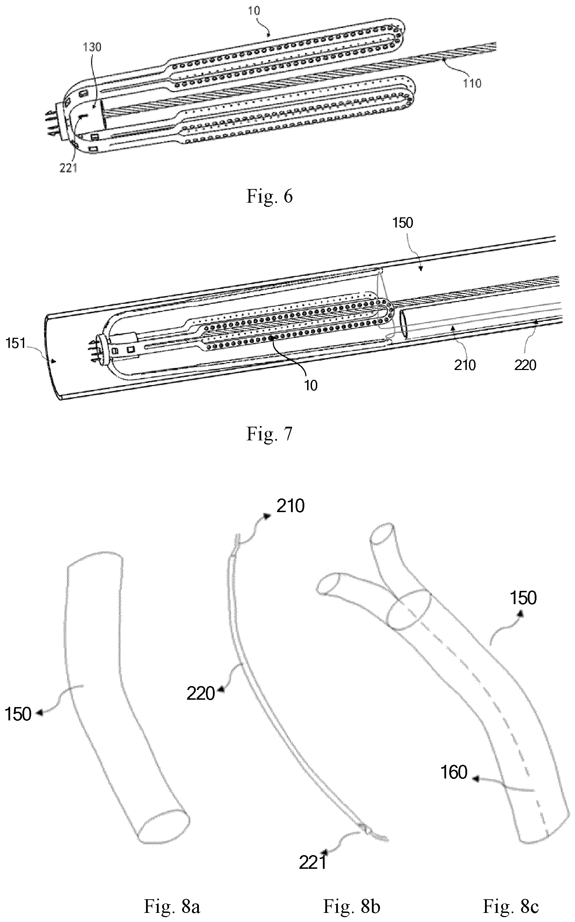

In a specific embodiment, the connector 130 is a hollow tubular structure comprising a hollow cavity 131, which allows the connector 130 to be sleeved over the base 20. The connector 130 comprises engagement slots 132 in its side wall, into which the elastic engagement elements 221 can engage. The engagement slots 132 are provided in correspondence with the respective elastic engagement elements 221. Preferably, the connector 130 is a thin-wall cylindrical structure optionally having an inner diameter of 3.5-8 mm and a wall thickness of 0.5-1.5 mm. More specifically, referring to Fig. 6, the elastic engagement elements 221 can extend from the receptacles 201 in the side face of the base 20 into the respective engagement slots 132 in the connector 130 and then function as stop blocks to prevent separation of the ventricular function assisting device from the delivery rod 110 during delivery.

Preferably, the delivery rod 110 includes a handle (not shown) at a proximal end thereof, which can be manipulated to rotate, push or pull the delivery rod 110, thereby advancing, retracting or alter the orientation of the ventricular function assisting device. Additionally, upon a failure in release, the delivery rod 120 can also be used as a pushrod to achieve retrieval of the ventricular function assisting device. The delivery rod 120 can be used in cooperation with the retrieval device to inwardly collapse the ventricular function assisting device. The delivery rod 110 is a hollow tubular structure allowing passage of a guidewire therethrough. For example, the delivery rod 110 may be a coil spring structure in the form of a hollow tube, which is made of one or more helically wound wires. The coil spring structure allows the delivery rod 110 to have better bendability and facilitates its passage through various complex structures within the heart. Preferably, the delivery rod 110 has different stiffness across its own length. For example, it may consist of differently stiff sections, which are joined together. Optionally, the delivery rod 110 may be least stiff at the middle. For example, it may include an intermediate section made of a flexible material or structure, which makes the intermediate section easier to flex. Alternatively, the intermediate section of the delivery rod 110 may be implemented as an easily bendable coiled tube. In a preferred embodiment, the delivery rod 110 includes distal section, an intermediate section and a proximal section, which are joined end-to-end sequentially in this order along its axis. The proximal and distal sections are stiffer than the intermediate section, making the intermediate section easier to bend. The proximal and distal sections may be both made of harder materials, which ensure good pushability of the proximal section and good manipulability of the distal section as required by retrieval.

Referring to Fig. 7, the delivery device further includes a delivery sheath 150, and the ventricular function assisting device can be crimped into the delivery sheath 150 and delivered therein to a target site. Moreover, the delivery rod 110 may be used to manipulate the ventricular function assisting device in the delivery sheath 150 to achieve its release, retrieval or the like.

Referring back to Figs. 4a and 4b, preferably, the ventricular function assisting device further includes an anchor structure 30 attached to the base 20. The anchor structure 30 may be located at the most distal position of the ventricular function assisting device. The base 20 and the anchor structure 30 may be integrally formed, or separately fabricated and then coupled to each other. The anchor structure 30 is configured for attachment to (target) apical tissue, dispensing with the need to arrange micro-spikes, barbs or other attachment structures on the backbones 11, which may cause greater damage to the cardiac tissue during their attachment, and attachment of which requires more difficult control. In a specific embodiment, the anchor structure 30 includes a holder and multiple spike-like structures 31 provided on the holder. The spike-like structures 31 extend toward the outside of the support structure 10 and are configured to pierce apical tissue, thereby anchoring the device to ventricular tissue. The holder of the anchor structure 30 is connected to the base 20, for example, by welding, adhesive bonding, integral formation, or the like. The anchor structure 30 comprises an anchor bore 32 allows passage of a guidewire therethrough. The anchor bore 32 may have a diameter matching a diameter of the guidewire, such as 1-3 mm. In order to achieve more secure anchoring, the spike-like structures 31 are preferred to each have a broadened tip. The spike-like structures 31 are desired not to be too long or too short. An excessive length may lead to complete piercing and perforation of the ventricle, while an insufficient length cannot ensure secure anchoring. Generally, each spike-like structure 31 has a length not exceeding a thickness of the inner ventricular wall, especially a thickness thereof at the apex. If required, any suitable number of spike-like structures 31 may be included. Preferably, 3-8 spike-like structures 31 are included, and each has a length of 3-22 mm. The spike-like structures 31 may be separatedly fabricated, and then fixed to the holder. Further, they may be made of a biocompatible metal material, or a degradable material.

Referring back to Fig. 7, the delivery sheath 150 preferably has a flexible distal section 151 located at the distal end, which is more flexible than the remaining of the delivery sheath 150, allowing easier loading and release of the ventricular function assisting device when loading and releasing of the ventricular function assisting device. In general, the ventricular function assisting device is crimped into the flexible distal section 151. In practical fabrication, the increased flexibility of the flexible distal section 151 can be achieved by virtue of its material and/or structure. For example, it may be implemented as a slender hose, or a tube with axially extending slits, which can be narrowed or widened to cause the tube to shrink or stretch. Alternatively, the tube may be formed of a less stiff material. Many other approaches are possible, and the present application is not limited to any particular approach. In the folded configuration, the ventricular function assisting device may be inserted into the flexible distal section 151 through an opening of distal end of the delivery sheath 150. The delivery sheath 150 usually has an inner diameter of 15-18 Fr and a length comparable to that of the delivery rod 110.

The delivery and retrieval system further includes a retrieval device, which is used in cooperation with the delivery device to partially or completely retrieve the ventricular function assisting device. Specifically, the retrieval device includes a constricting mechanism configured to be releasably connected to the support structure 10. The delivery rod 110 and the constricting mechanism can be used in cooperation with each other to load the ventricular function assisting device into the delivery sheath 150.

Referring to Figs. 7 and 8a to 8c, the constricting mechanism preferably includes a pull thread 210 having a first end, a second end and a thread body extending between the first and second ends. The thread body is configured to successively pass through the threading holes in the backbones 11 and then to extend axially through the interior of the delivery sheath 150. Moreover, the first and second ends are locked at a proximal end of the delivery sheath 150. With this arrangement, the pull thread 210 can be manipulated to gather a proximal end of the support structure 10 and to pull the gathered support structure 10 into the delivery sheath 150. Retrieval can be accomplished only by cooperation of the pull thread 210 with the delivery rod 110. The ventricular function assisting device may be first held stationary by pushing the delivery rod 110 against the base 20, and the pull thread 210 may be then manipulated to gather the proximal end of the support structure 10. After sufficient gathering is attained, the delivery rod 110 and the pull thread 210 may be simultaneously operated to load the ventricular function assisting device into the delivery sheath 150.

Retrieving the ventricular function assisting device using the pull threads 210 and the threading holes allows the delivery device to have a reduced size because the pull thread 210 is small in size and flexible. Moreover, this can ensure desired compliance of the delivery device, which facilitates withdrawal and retrieval. The pull thread 210 is desired to be longer than the delivery sheath 150 and long enough to extend to the distal end of the delivery sheath 150 and then back to and beyond the proximal end of the delivery sheath 150. In addition to pulling and gathering the ventricular function assisting device and retrieving it into the delivery sheath 150, the pull thread 210 can also be used to slow down expansion of the backbones 11 after the ventricular function assisting device is released distally from the delivery sheath 150, avoiding the backbones 11 from possibly causing damage to ventricular tissue due to otherwise too fast self-expansion. When the ventricular function assisting device is delivered in the delivery sheath 150 to a target site, the pull thread 210 can be manipulated to achieve either complete release and unfolding of the backbones 11, or partial release thereof from the delivery sheath 150.

Optionally, the retrieval device may further include a retrieval catheter 220 disposed within the delivery sheath 150 and is arranged in parallel to the delivery rod 110. That is, it is not the case that one of the delivery rod 110 and the retrieval catheter 220 is received in the other. The retrieval catheter 220 is configured to receive the pull thread 210, avoiding its winding on the delivery rod 110, which may add difficulties to retrieval. Of course, in other embodiments, the retrieval catheter 220 may be omitted. Further, proximal end of the retrieval catheter 220 may include attachment structures, to which the ends of the pull thread 210 are attached. In some embodiments, the delivery rod 110 and the retrieval catheter 220 are disposed in the same lumen of the delivery sheath 150. In some other embodiments, the delivery rod 110 and the retrieval catheter 220 are disposed in two independent lumens of the delivery sheath 150, as shown in Fig. 8c. When the delivery sheath 150 comprises two lumens, a first rapid exchange port 160 is preferably provided between the lumens. The first rapid exchange port 160 may extend a certain length along an axis of the delivery sheath 150. The pull thread 210 or other necessary components may be passed through the first rapid exchange port 160 to speed up operation and increase surgical efficiency.

In this embodiment, the retrieval catheter 220 may comprise a single lumen, or two separate lumens extending along its axis. In the latter case, separate sections of the pull thread 210 may be passed through the respective lumens of the retrieval catheter 220 in order to avoid twisting, knotting or other undesired conditions that may occur to the thread sections as a result of pulling the pull thread 210, allowing for safer and more reliable retrieval. It will be understood that one of the two sections of the pull thread 210 extends from the distal end of the delivery sheath 150 back to the proximal end thereof after the thread has been passed through the threading holes, and the other section extends from the proximal end of the delivery sheath 150 to the distal end thereof before the thread is passed through the threading holes.

Referring to Fig. 8b, a second rapid exchange port 221 may be provided on a side wall of the retrieval catheter 220. The second rapid exchange port 221 may be, for example, square in shape, and preferably extends a certain length along an axis of the retrieval catheter 220. The second rapid exchange port 221 provided in the side wall of the retrieval catheter 220 can speed up operation of the pull thread 210 and allows for more convenient surgical operation. Preferably, the proximal end of the retrieval catheter 220 include two tightening valves (not shown) serving as the aforementioned attachment structures that the ends of the pull thread 210 are attached to. In this way, the pull thread 21 can be pulled by retracting the retrieval catheter 220, facilitating power transmission and making the ventricular function assisting device easier to retrieve.

In this embodiment, there is also provided a preferred method of implanting the ventricular function assisting device as defined above, for example, into a left ventricle. It includes the steps as follows:
Step 1: Couple the ventricular function assisting device to the delivery rod 110, pass the pull thread 210 of the retrieval device successively through the threading holes at the proximal ends of the ventricular function assisting device and manipulate the delivery rod 110 in cooperation with the pull thread 210 to load the ventricular function assisting device into the delivery sheath 150.
Step 2: Advance the ventricular function assisting device, together with the delivery rod 110 and the retrieval device, within the delivery sheath 150 to a target site of the left ventricle.
Step 3: Release the ventricular function assisting device and determine whether it is successfully released. For example, determinations may be made as to whether it is released at the target site, whether the backbones 11 have completely expanded, whether the backbones 11 are pressed against an inner wall of the ventricle in an undesirable manner, etc.

If the ventricular function assisting device is successfully released, the following step is performed:
Step 4a: Detach the pull thread 210 and the retrieval catheter 220, decouple the delivery rod 110 from the ventricular function assisting device, and withdraw all the other component than the ventricular function assisting device from the patient's body.

If the ventricular function assisting device is not successfully released, the following step is performed:
Step 4b: Hold the ventricular function assisting device stationary by pushing the delivery rod 110 against it, and simultaneously retract the retrieval catheter 220 and hence the pull thread 210 to gather the proximal ends of the backbones 11 toward the longitudinal axis of the base 20. The folded the ventricular function assisting device is then pulled back into the delivery sheath 150, i.e., re-loaded therein. After the reloading is completed, another release attempt may be made, or all the components may be withdrawn from the patient's body.

In step 1, the ventricular function assisting device is coupled to the delivery rod 110 by engaging the connector 130 with the locking mechanism 22 of the base 20. Moreover, the pull thread 210 is successively passed through the threading holes at the proximal ends of the backbones 11 and then extends from the distal end of the delivery sheath 150 to and beyond the proximal end of the retrieval catheter 220. The ends of the pull thread 210 are then attached to the tightening valves (not shown) at the proximal end of the retrieval catheter 220. After that, the delivery rod 110 is manipulated in cooperation with the pull thread 210 to fold the ventricular function assisting device and load it into the flexible distal section 151 of the delivery sheath 150.

In step 2, before the ventricular function assisting device is implanted, a puncture is made in the femoral vein, and a leading end of the guidewire is inserted into the femoral vein and advanced through the inferior vena cava into the patient's left ventricle. After that, the delivery sheath 150, together with the ventricular function assisting device and the retrieval device loaded therein, is delivered on the guidewire to a target site of the left ventricle.

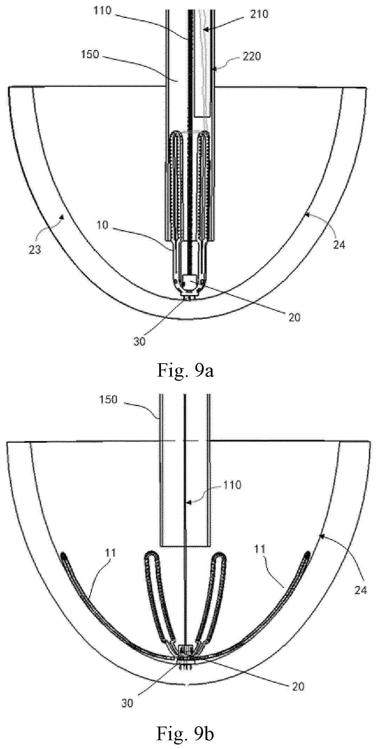

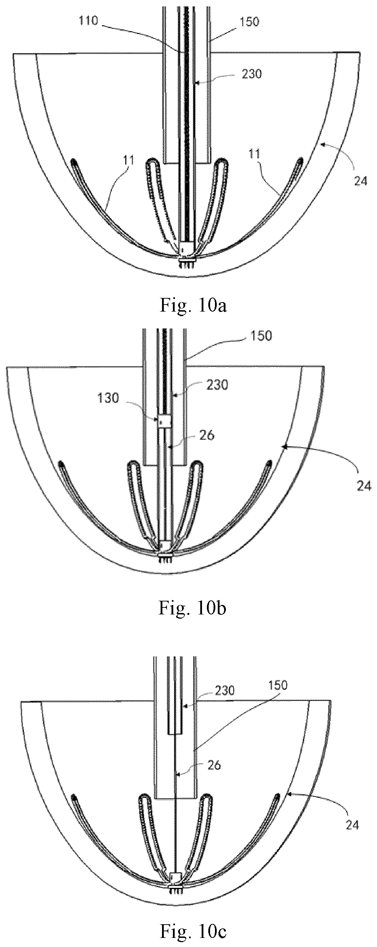

Delivery and release of the ventricular function assisting device are described in greater detail below with reference to Figs. 9a to 9b and Figs. 10a to 10d.

Referring to Fig. 9a, once the distal end of the delivery sheath 150 reaches the target site of the left ventricle, the delivery rod 110 is pushed toward the distal end of the delivery sheath 150 to gradually release the ventricular function assisting device from the distal end of the delivery sheath 150.

Referring to Fig. 9b, after the ventricular function assisting device is completely pushed out of the delivery sheath 150, the backbones 11 will gradually unfold radially due to their super-elasticity or shape memory until they are pressed against an inner wall 24 of the ventricle. In this process, the unfolding of the backbones 11 can be slowed down by pulling the pull thread 210 in order to avoid the backbones 11 from possibly causing damage to ventricular tissue 23 due to otherwise too fast self-expansion. If the release succeeds, the delivery rod 110 is pushed to cause the spike-like structures 31 at the bottom of the anchor structure 30 to pierce apical ventricular tissue 23, anchoring the device at the apex. If release at a undesired location, insufficient pressing of the backbones 11 against the inner ventricular wall 24 or another undesirable condition occurs, the pull thread 210 may be pulled and the delivery rod 110 may be manipulated in concert to retrieve the ventricular function assisting device back into the delivery sheath 150. Thereafter, the delivery sheath 150 may be rotated or otherwise operated to relocate the distal end of the delivery sheath 150 and hence the ventricular function assisting device, where another attempt may be made by the delivery rod 110 to release the ventricular function assisting device.

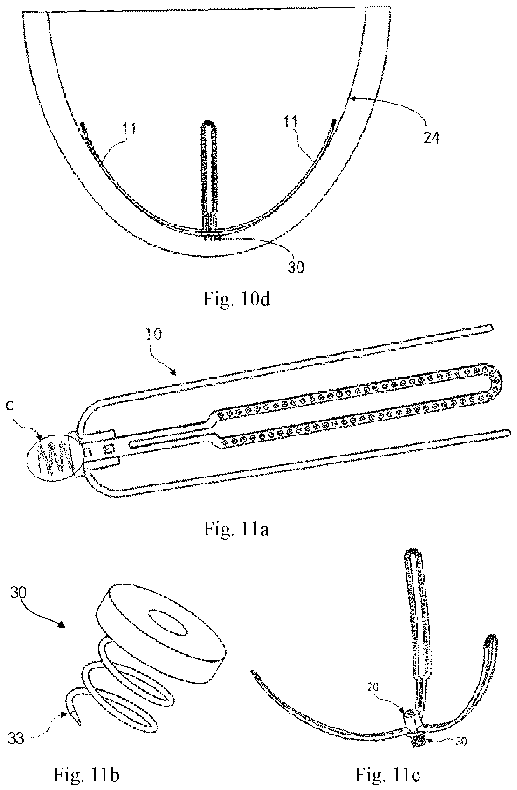

In a non-limiting embodiment, after the ventricle assisting device is successfully released, an additional catheter 230 may be used to decouple the delivery rod 110 from the ventricular function assisting device, as shown in Figs. 10a to 10d. Preferably, the additional catheter 230 forms part of the delivery device. It is configured to be sleeved over the exterior of the delivery rod 110 to decouple the delivery rod 110 from the base 20 by disengaging the locking mechanism 22 from the connector 130. In a specific embodiment, the additional catheter 230 has an inner diameter slightly greater than an outer diameter of the distal connector 130 of the delivery rod 110. Therefore, the connector 130 can be received into the additional catheter 230. Reference is made to Figs. 10a and 10b for more details of the decoupling process. First of all, the additional catheter 230 is advanced on the guidewire 26 into the left ventricle until the connector 130 of the delivery rod 110 is completely received into the additional catheter 230. In this process, an inner wall of the additional catheter 230 radially compresses the elastic engagement elements 221 (e.g., leaf spring tabs) completely into the receptacles 201, disengaging the delivery rod 110 from the ventricle assisting device and making it possible to remove the delivery rod 110 simply by retracting proximally. After the delivery rod 110 is withdrawn, the other components than the ventricular function assisting device may be successively withdrawn, as shown in Figs. 10c and 10d.

### <Embodiment 2>

A second embodiment of the present invention is described below, with the emphasis of description being essentially placed on its differences from the first embodiment. Any common feature shared these embodiments will not be repeated below, and reference can be made to the above description of the first embodiment.

Referring to Figs. 11a to 11c, differing from the first embodiment, the anchor structure 30 in Embodiment 2 includes a holder and a helical structure 33 disposed on the holder. The helical structure 33 is configured to be screwed into an inner ventricular wall 24, thereby anchoring the device to ventricular tissue 23. As noted above, in Embodiment 1, the anchor structure 30 achieves this by piercing of the inner ventricular wall 24 by the spike-like structures 31.

Similarly, the helical structure 33 may be made of a biocompatible metal or degradable material. Preferably, the helical structure 33 has a diameter of 1-5 mm and is fabricated from a wire with a diameter of 0.2-0.5 mm. Moreover, a length of the helical structure is configured to prevent it from screwing through the ventricular tissue 23, and preferably 4-20 mm. According to Embodiment 2, the ventricular function assisting device can be more securely attached to the ventricular tissue 23. Optionally, the helical structure 33 may be made of a biodegradable material.

### <Embodiment 3>

A third embodiment of the present invention is described below, with the emphasis of description being essentially placed on its differences from the first embodiment. Any common feature shared these embodiments will not be repeated below, and reference can be made to the above description of the first embodiment. Differing from the first embodiment, the backbones 11 in Embodiment 3 are mesh-like stent structures, i.e., implemented as a stent-like structure. Mesh openings in the mesh-like stent structures form hollow slots, and the drug reservoirs 115 are formed in surfaces of struts of the mesh-like stent structures.

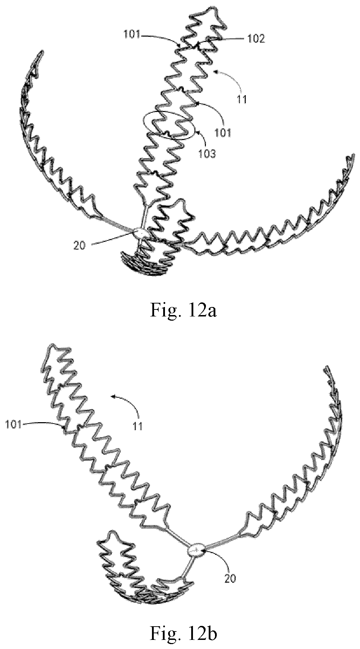

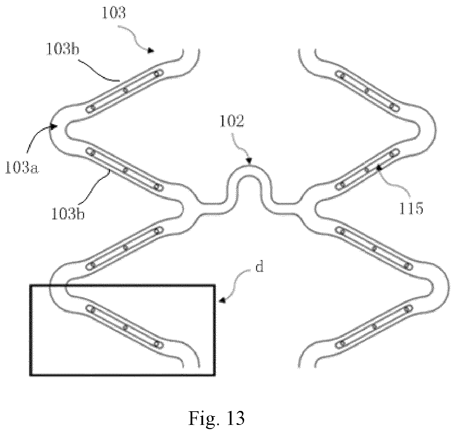

Referring to Figs. 12a, 12b and 13, in specific embodiments, each backbone 11 is a single-layer mesh sheet structure including a plurality of wavy sections 101 arranged side-by-side and circumferentially around the base 20. For each backbone, first ends of the wavy sections 101 are joined and then are coupled to the base 20. The second ends of the wavy sections 101 are joined together to form the free end 113 of the backbone 11, and any pair ofwavy sections 101 is connected by deformable connecting struts 102 to enhance stability of the structure. Additionally, these connecting struts 102 delimit many hollow slots in the backbones 11. Each backbone 11 may include two or more wavy sections 101, preferably two wavy sections 101 according to embodiments of the present invention. The connecting struts 102 may be stretchable and deformable and of various shapes. Generally, they are in the shape of polylines or curved lines, such as N-shaped lines. Alternatively, they may also be S-shaped, Z-shaped or of any other suitable shape.

Referring to Fig. 13, each wavy section 101 may include a plurality of repetitive units 103 each consisting of a curved section 103a and two strut sections 103b joined to respective ends of the curved section 103a. The drug reservoirs 115 are provided in outer surfaces of the strut sections 103b. The strut sections 103b may be wider and/or thicker than the connecting struts 102. Due to small dimensions of the wavy sections 101, the drug reservoirs 115 are formed in the strut sections 103b usually by laser engraving. The drug reservoirs 115 may have a length smaller than or equal to a length of the strut sections 103b. Without limitation, the strut sections 103 may be straight or curved.

According to Embodiment 3, constructing the backbones as a mesh-like stent structure can significantly reduce the weight of the ventricular function assisting device, as well as the burden on the heart. Moreover, this improves adhesion of the inner ventricular wall 24 to the inner wall of the ventricle and long-term stability of adhesion. Moreover, the backbones 11 are made more laterally flexible and hence less stressed during operation, in particular as the ventricle twists or moves longitudinally. If required, any number of backbones 11 may be included. For example, in one embodiment, as shown in Fig. 12a, four backbones 11 are included and uniformly arranged circumferentially. In another exemplary embodiment, as shown in Fig. 12b, three backbones 11 are included and uniformly arranged circumferentially.

In this application, there is also provided a ventricular function assisting system including the ventricular function assisting device and the delivery and retrieval system according to any of the foregoing embodiments.

In summary, the ventricular function assisting device provided by the present invention, on one hand, utilizes the self-expanding force of the backbones to press against the inner wall of the ventricle, thereby enhancing ventricular function during diastole and helping the stiff and non-compliant ventricle to properly relax and fill. On the other hand, a drug can be contained in the drug reservoirs in the outer surfaces of the backbones, instead of being coating on the inner and outer surfaces of the backbones. With this arrangement, the drug can be released from the drug reservoirs essentially toward the inner wall of the ventricle, providing for targeted delivery of the drug. This allows the drug to be loaded in a reduced amount and prevents its diffusion in all directions, which may adversely affect the growth of ventricular tissue. In addition to this, growth and spreading of ventricular tissue on the ventricular function assisting device can be facilitated, enhancing post-implantation adhesion of the ventricular function assisting device to the inner wall of the ventricle and long-term stability of the adhesion.

It will be understood that features of the present invention have been disclosed in the foregoing preferred embodiments to provide a better understanding of the invention to those skilled in the art. It would be appreciated by those skilled in the art that, on the basis of the disclosure herein, it would be easy to modify the present invention while still achieving the same objects and/or advantages as the embodiments disclosed herein. Those skilled in the art would also recognize that such similar configurations do not depart from the scope of disclosure of this invention and could be subject to various changes, substitutions, and alterations without departing the scope of disclosure of the invention.

## Claims

1. A ventricular function assisting device, comprising a folded configuration and an expanded configuration and being switchable between the folded configuration and the expanded configuration, wherein the ventricular function assisting device comprises a support structure and a base, wherein the base is configured to be releasably connected to a delivery device, wherein the support structure comprises a plurality of backbones which are arranged sequentially and circumferentially around the base, wherein a first end of each backbone is connected to the base and the second end of each backbone is a free end, and wherein outer surfaces of at least some of the backbones are provided with drug reservoirs.

2. The ventricular function assisting device according to claim 1, wherein the drug reservoir comprises a large-diameter hole and a small-diameter hole in communication with each other, wherein the large-diameter hole has a larger diameter than the small-diameter hole, wherein the large-diameter hole extends through an outer surface of the backbone, wherein the small-diameter hole extends through an inner surface of the backbone, and wherein the large-diameter hole is configured to contain a drug therein.

3. The ventricular function assisting device according to claim 1 or 2, wherein at least some of the backbones comprise hollow areas, and wherein at least some of the backbones are provided with the drug reservoirs in solid areas of the backbones outside the hollow areas.

4. The ventricular function assisting device according to claim 3, wherein the hollow area is made up of a single continuous hollow slot and/or a plurality of discontinuous hollow slots.

5. The ventricular function assisting device according to claim 4, wherein the backbone is in a form of a sheet or a bar, and a middle portion of the backbone is formed with a single continuous hollow slot extending from a first end to a second end, and wherein the drug reservoir is provided along a solid area that is outside of the single continuous hollow slot.

6. The ventricular function assisting device according to claim 4, wherein the backbone is a mesh-like stent structure, wherein the mesh openings in the mesh-like stent structure form the hollow slots, and wherein the drug reservoirs are provided in surfaces of struts in the mesh-like stent structure.

7. The ventricular function assisting device according to claim 6, wherein the mesh-like stent structure is a single-layer mesh sheet structure comprising a plurality of wavy sections that are arranged circumferentially and side-by-side around the base, wherein, for each backbone, first ends of the wavy sections are joined and then connected to the base, and second ends of the wavy sections are joined to form the free end of the backbone, and wherein any pair of the wavy sections is connected by at least one deformable connecting strut.

8. The ventricular function assisting device according to claim 7, wherein each wavy section comprises a plurality of repetitive units, wherein each repetitive unit consists of a curved section and strut sections, wherein each end of the curved section is joined to the strut section, wherein each drug reservoir is provided in an outer surface of the strut section and has a length smaller than or equal to a length of the strut section.

9. The ventricular function assisting device according to claim 1 or 2, wherein a threading hole is provided at the free end of each backbone, wherein the threading hole extends through the drug reservoir, and/or wherein the threading hole are arranged independently from the drug reservoirs.

10. The ventricular function assisting device according to claim 1 or 2, further comprising an anchor structure attached to the base, wherein the anchor structure is configured to connect a target tissue.

11. The ventricular function assisting device according to claim 10, wherein the anchor structure comprises a plurality of spike-like structures extending toward an outside of the support structure, wherein the spike-like structures are configured to pierce the target tissue, or wherein the anchor structure comprises a helical structure configured to be screwed into the target tissue.

12. The ventricular function assisting device according to claim 10, wherein a portion of the anchor structure, which is configured to be inserted into the target tissue, is made of a biodegradable material.

13. The ventricular function assisting device according to claim 1 or 2, wherein the base is provided with a locking mechanism configured for a snap engagement with the delivery device.

14. The ventricular function assisting device according to claim 13, wherein the locking mechanism comprises a plurality of elastic engagement elements, wherein the plurality of elastic engagement elements are arranged sequentially and circumferentially around the base, wherein each elastic engagement element is configured to be retracted into the base under an action of an external force applied thereto and to extend out of the base due to an elasticity thereof when the external force is removed.

15. The ventricular function assisting device according to claim 1 or 2, further comprising a drug structure that is provided in the drug reservoir, wherein the drug structure is comprised of a drug and a polymer carrier.

16. The ventricular function assisting device according to claim 1 or 2, wherein each backbone is elastic, and/or an end surface at the free end of each backbone is a smooth curved surface.

17. The ventricular function assisting device according to claim 1 or 2, wherein a maximum diameter of the support structure after expanded is greater than an inner diameter of a portion of a ventricle where the support structure presses against an inner wall of the ventricle , and thereby supporting the support structure on the inner wall of the ventricle by virtue of a stretchability thereof.

18. The ventricular function assisting device according to claim 1 or 2, comprising three or four backbones, wherein the backbones are uniformly arranged circumferentially around the base.

19. A delivery and retrieval system for delivering and retrieving the ventricular function assisting device of any one of claims 1 to 18, comprising a retrieval device and a delivery device, wherein the retrieval device comprises a constricting mechanism, wherein the delivery device comprises a delivery rod and a delivery sheath, wherein a distal end of the delivery rod is configured to be releasably connected to the base of the ventricular function assisting device, wherein the constricting mechanism is configured to be releasably connected to the support structure of the ventricular function assisting device, wherein the delivery rod and the constricting mechanism are configured to cooperate with each other to load the ventricular function assisting device into the delivery sheath and/or to unload the ventricular function assisting device from the delivery sheath.

20. The delivery and retrieval system according to claim 19, wherein a free end of each backbone is provided with a threading hole, wherein the constricting mechanism comprises a pull thread configured to be successively passed through each threading hole of the backbones and extend axially within the delivery sheath, and wherein two ends of the pull thread extend beyond a proximal end of the delivery sheath and are fixed.

21. The delivery and retrieval system according to claim 20, wherein the retrieval device further comprises a retrieval catheter disposed in the delivery sheath, wherein the retrieval catheter is arranged in parallel to the delivery rod within the delivery sheath, wherein the pull thread is passed through the retrieval catheter, and a proximal end of the retrieval catheter is provided with attachment structures, and wherein the attachment structures are configured to fix two ends of the pull thread.

22. The delivery and retrieval system according to claim 21, wherein the delivery rod and the retrieval catheter are disposed in a single lumen of the delivery sheath or different lumens of the delivery sheath.

23. The delivery and retrieval system according to claim 21, wherein the retrieval catheter comprises two separate axially-extending lumens, and wherein two sections of the pull thread are passed through two lumens, respectively.

24. The delivery and retrieval system according to claim 19, wherein the delivery device further comprises an additional catheter disposed in the delivery sheath, wherein the additional catheter is configured to be sleeved over the exterior of the delivery rod and thereby decouple the delivery rod from the base.

25. The delivery and retrieval system according to claim 24, wherein the base is provided with a locking mechanism, wherein a distal end of the delivery rod is provided with a connector, and wherein the connector is configured for a snap engagement with the locking mechanism.

26. The delivery and retrieval system according to claim 25, wherein the connector is a hollow tubular structure and is configured to be sleeved over the base.

27. The delivery and retrieval system according to claim 19, wherein the delivery rod is a coil spring formed by helically winding one or more wires, and/or wherein the delivery rod has different stiffness along an axial direction thereof, wherein an intermediate section of the delivery rod is less stiff than a proximal section and a distal section of the delivery rod.

28. The delivery and retrieval system according to claim 19, wherein the delivery sheath has a flexible distal section located at a distal end, wherein the flexible distal section is configured to receive the ventricular function assisting device therein.

29. A ventricular function assisting system, comprising the ventricular function assisting device of any one of claims 1 to 18 and the delivery and retrieval system of any one of claims 19 to 28.
